# EUROPEAN PATENT SPECIFICATION

(11) **EP 2 542 168 B1**
(45) Date of publication and mention of the grant of the patent: **06.05.2015**
(21) Application number: 11751285.5
(22) Date of filing: 02.03.2011
(51) Int. Cl.: A61B 17/70, A61B 17/84, A61B 17/86, A61F 2/44

(54) **DYNAMIC VERTEBRAL CONSTRUCT**
DYNAMISCHES BANDSCHEIBENKONSTRUKT
CONSTRUCTION VERTÉBRALE DYNAMIQUE

(30) Priority: 03.03.2010 US 716746
(43) Date of publication of application: 09.01.2013
(73) Proprietor: Warsaw Orthopedic, Inc., Warsaw, IN 46581 (US)
(72) Inventor: BONIN, Henry, K., Memphis Tennessee 38107 (US); MARIK, Greg, C., Collierville Tennessee 38017 (US); CARLS, Thomas, A., Memphis Tennessee 38103 (US)
(74) Representative: Viering, Jentschura & Partner Patent- und Rechtsanwälte
(86) International application number: PCT/US2011/026864
(87) International publication number: WO 2011/109513

(56) References cited:
- US-A- 5 382 248
- US-A- 5 395 371
- US-A- 5 562 737
- US-A1- 2005 033 297
- US-A1- 2005 033 432
- US-A1- 2009 171 395
- US-B1- 6 585 738
- US-B2- 7 608 096

## Description

The invention relates to a vertebral construct according to the preamble of claim 1.

### BACKGROUND

A vertebral construct according to the initially mentioned type is known from US 2005/0033432 A1 discloses. Further vertebral constructs are known from, e.g., US 5,395,371, US 2009/0171395 A1, US 5,562,737 and US 2006/0064088 A1.

Spinal disorders such as degenerative disc disease, disc herniation, osteoporosis, spondylolisthesis, stenosis, scoliosis and other curvature abnormalities, kyphosis, tumor, and fracture may result from factors including trauma, disease and degenerative conditions caused by injury and aging. Spinal disorders typically result in symptoms including pain, nerve damage, and partial or complete loss of mobility.

Non-surgical treatments, such as medication, rehabilitation and exercise can be effective, however, may fail to relieve the symptoms associated with these disorders. Surgical treatment of these spinal disorders include discectomy, laminectomy, fusion and implantable prosthetics. As part of these surgical treatments, spinal constructs such as vertebral rods are often used to provide stability to a treated region. Rods redirect stresses away from a damaged or defective region while healing takes place to restore proper alignment and generally support the vertebral members. During surgical treatment, one or more rods may be attached via fasteners to the exterior of two or more vertebral members. This disclosure describes an improvement over these prior art technologies.

### SUMMARY

Accordingly, a vertebral construct is provided, which includes a spinal rod connected to at least one fastening element in a configuration that facilitates relative dynamic translation.

In accordance with the principles of the present disclosure, a vertebral construct according to claim 1 is provided.

A further embodiment is described in dependent claim 2.

### BRIEF DESCRIPTION OF THE DRAWINGS

Figs. 8-14 do not form embodiments of the invention as such, but help explaining aspects of the invention.

The present disclosure will become more readily apparent from the specific description accompanied by the following drawings, in which:
FIG. 1 is a plan view of one particular embodiment of a vertebral construct in accordance with the principles of the present disclosure;
FIG. 2 is a side view of the vertebral construct shown in FIG. 1;
FIG. 3 is a perspective view of the vertebral construct shown in FIG. 1 with a portion of a fastening element removed;
FIG. 4 is a side view of the vertebral construct shown in FIG. 1;
FIG. 5 is a side view of the vertebral construct shown in FIG. 1;
FIG. 6 is a side view of vertebrae having a spinal disorder;
FIG. 7 is a side view of the vertebral construct shown in FIG. 1 attached with the vertebrae shown in FIG. 6;
FIG. 8 is a perspective view of a vertebral construct in accordance with the principles of the present disclosure;
FIG. 9 is a perspective view of the vertebral construct shown in FIG. 8 with a damping element removed;
FIG. 10 is a perspective view of a fastening element of the vertebral construct shown in FIG. 8;
FIG. 11 is a perspective view of a vertebral construct in accordance with the principles of the present disclosure with a damping element removed;
FIG. 12 is a perspective view of a vertebral construct in accordance with the principles of the present disclosure with a damping element removed;
FIG. 13 is a perspective view of a fastening element of the vertebral construct shown in FIG. 12;
FIG. 14 is a perspective view of a vertebral construct in accordance with the principles of the present disclosure; and
FIG. 15 is a perspective view of one embodiment of the vertebral construct in accordance with the principles of the present disclosure.

Like reference numerals indicate similar parts throughout the figures DETAILED DESCRIPTION

The exemplary embodiments of the vertebral construct and methods of use disclosed are discussed in terms of medical devices for the treatment of spinal disorders and more particularly, in terms of a vertebral construct including a spinal rod connected to at least one fastening element in a configuration that facilitates relative dynamic translation. It is envisioned that the vertebral construct and methods of use disclosed provide stability and maintains structural integrity while reducing stress on spiral elements. It is envisioned that the present disclosure may be employed to treat spinal disorders such as, for example, degenerative disc disease, disc herniation, osteoporosis, spondylolisthesis, stenosis, scoliosis and other curvature abnormalities, kyphosis, tumor and fractures. It is further envisioned that the present disclosure may be employed with surgical treatments including open surgery and minimally invasive procedures, of such disorders, such as, for example, discectomy, laminectomy, fusion, bone graft and implantable prosthetics. It is contemplated that the present disclosure may be employed with other osteal and bone related applications, including those associated with diagnostics and therapeutics. It is further contemplated that the disclosed vertebral construct may be employed in a surgical treatment with a patient in a prone or supine position, employing a posterior, lateral or anterior approach. The present disclosure may be employed with procedures for treating the lumbar, cervical, thoracic and pelvic regions of a spinal column. The system and methods of the present disclosure may also be used on animals, bone models and other non-living substrates, such as for training, testing and demonstration.

The present invention may be understood more readily by reference to the following detailed description of the invention taken in connection with the accompanying drawing figures, which form a part of this disclosure. It is to be understood that this invention is not limited to the specific devices, methods, conditions or parameters described and/or shown herein, and that the terminology used herein is for the purpose of describing particular embodiments by way of example only and is not intended to be limiting of the claimed invention. Also, as used in the specification and including the appended claims, the singular forms "a," "an," and "the" include the plural, and reference to a particular numerical value includes at least that particular value, unless the context clearly dictates otherwise. Ranges may be expressed herein as from "about" or "approximately" one particular value and/or to "about" or "approximately" another particular value. When such a range is expressed, another embodiment includes from the one particular value and/or to the other particular value. Similarly, when values are expressed as approximations, by use of the antecedent "about," it will be understood that the particular value forms another embodiment.

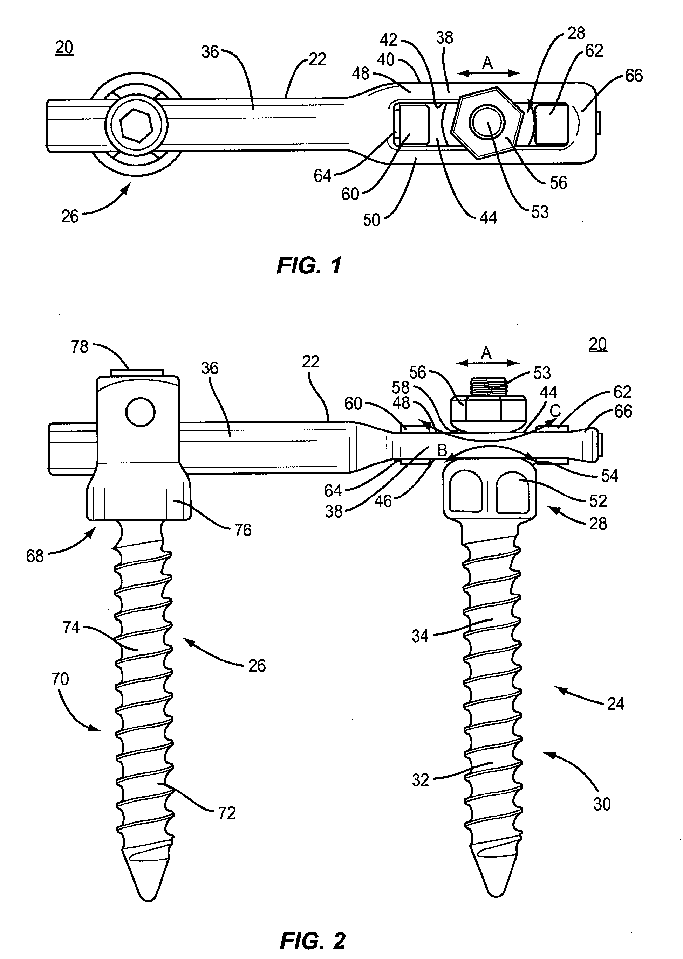

The following discussion includes a description of a vertebral construct, related components and exemplary methods of employing the vertebral construct in accordance with the principles of the present disclosure. Alternate embodiments are also disclosed. Reference will now be made in detail to the exemplary embodiments of the present disclosure, which are illustrated in the accompanying figures. Turning now to FIGS. 1-5, there is illustrated components of a vertebral construct in accordance with the principles of the present disclosure.

The components of the vertebral construct are fabricated from materials suitable for medical applications, including metals, polymers, ceramics, biocompatible materials and/or their composites, depending on the particular application and/or preference of a medical practitioner. For example, the components of the vertebral construct, individually or collectively, can be fabricated from materials such as titanium, thermoplastics such as polyaryletherketone (PAEK) including polyetheretherketone (PEEK), polyetherketoneketone (PEKK) and polyetherketone (PEK), carbon-PEEK composites, PEEK-BaSO₄ polymeric rubbers, biocompatible materials such as polymers including plastics, metals, ceramics and composites thereof, rigid polymers including polyphenylene, polyamide, polyimide, polyetherimide, polyethylene, epoxy, and different components of the vertebral construct may have alternative material composites to achieve various desired characteristics such as strength, rigidity, elasticity, compliance, biomechanical performance, durability and radiolucency or imaging preference.

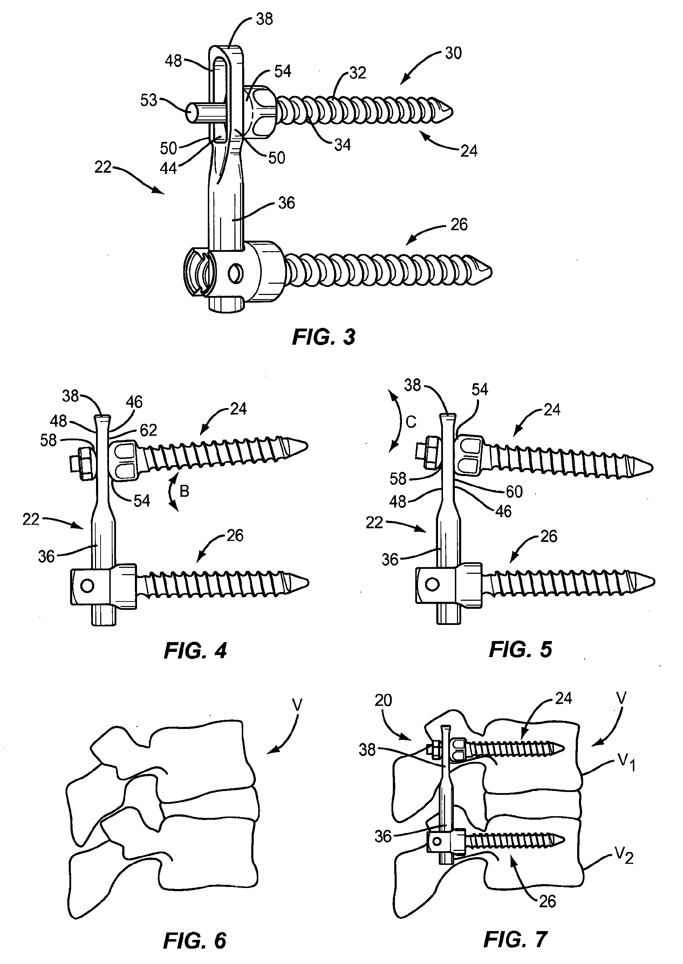

The vertebral construct is configured for attachment to vertebrae during surgical treatment of a spinal disorder, examples of which are discussed herein. A vertebral construct 20 includes a rod 22, and a first fastening element, such as, for example, a pedicle screw assembly 24 and a second fastening element, such as, for example, a pedicle screw assembly 26 that attach rod 22 to bony tissue, including vertebrae V (FIGS. 5 and 6), as will be discussed.

Pedicle screw assembly 24 has a first portion 28 and a second portion 30, which includes a threaded portion 32 of a screw 34. Threaded portion 32 is configured for engagement with tissue, such as, for example, fixation with vertebrae V. It is contemplated that second portion 30 may be non-threaded and attached with tissue via interference or friction fit, clips and/or barbs.

Rod 22 has a first portion, such as, for example, cylinder 36 and a second portion, such as, for example, band portion 38. Band portion 38 includes a wall 40 having an inner surface 42 that defines an elongated cavity, such as, for example, an elongated opening 44 configured to facilitate dynamic translation of first portion 28 therein relative to rod 22. First portion 28 translates with elongated opening 44, in the direction of arrows A shown in FIGS. 1 and 2.

Band portion 38 has an elongated plate configuration that defines a first face 46 configured to engage first portion 28 and an opposing second face 48 configured to engage an opposing portion of first portion 28. First face 46 and second face 48 are disposed about a perimeter of elongated opening 44. Band portion 38 includes bifurcated wall portions 50, which are separated and/or split to define elongated opening 44 in a configuration to capture first portion 28.

It is envisioned that the components of rod 22 may be monolithically formed, integrally connected or arranged with attaching elements. It is contemplated that cylinder 36 and band portion 38 can be variously dimensioned, for example, with regard to length, width, diameter and thickness. It is further contemplated that the respective cross-section of cylinder 36 and band portion 38, including wall 40, may have various configurations, for example, round, oval, rectangular, irregular, uniform and non-uniform. Cylinder 36 may have a different cross-sectional area, geometry, material or material property such as strength, modulus or flexibility relative to band portion 38. Faces 46, 48 may have a smooth, continuous surface, or alternatively, be textured or dimpled.

Cylinder 36 and band portion 38 are disposed along a longitudinal axis *a* of rod 22. Cylinder 36 may be angularly offset, perpendicular and/or staggered relative to band portion 38.

First portion 28 includes a portion of screw 34 including a head 52 having a spherical surface 54 engageable with first face 46 such that rod 22 is configured for multiple axis movement, along arrows B shown in FIGS. 2 and 4, relative to pedicle screw assembly 24. It is contemplated that such multiple axis movement includes rotation, in the direction shown by arrows B, of rod 22 relative to spherical surface 54. Head 52 has a threaded post 53 extending therefrom. First portion 28 also includes a nut 56 threaded onto post 53 to connect and movably capture rod 22 with pedicle screw assembly 24. Band portion 38 is movably attached with first portion 28 to facilitate relative translation and multiple axis movement, including rotation, therebetween, as described herein. Nut 56 is configured to prevent disassociation of rod 22 from pedicle screw assembly 24.

Nut 56 has a spherical surface 58 that is engageable with second face 48 such that rod 22 is configured for multiple axis movement, along arrows C shown in FIGS. 2 and 4, relative to pedicle screw assembly 24. It is contemplated that such multiple axis movement includes rotation, in the direction shown by arrows C, of rod 22 relative to spherical surface 58. Opposing faces 46, 48 of band portion 38 are engageable with opposing spherical surfaces 54, 58 such that rod 22 is configured for multiple axis movement, including rotation, relative to pedicle screw assembly 24. Engagement of opposing faces 46, 48 with opposing spherical surfaces 54, 58 facilitate bending of the vertebral bodies of vertebrae V, discussed below. Head 52 and nut 56 have a hexagonal configuration for engagement with a surgical tool to facilitate rotation and corresponding threaded fixation with vertebrae V. It is contemplated that faces 46, 48 may have alternate arcuate surfaces, angled surfaces, planar surfaces and/or undulating surfaces.

Pedicle screw assembly 26 has a first portion 68 fixedly connected to cylinder 36 and a second portion 70. Second portion 70 includes a threaded portion 72 of a screw 74. Threaded portion 72 is configured for engagement with tissue, such as, for example, fixation with vertebrae V. Rigid fixation of cylinder 36 to pedicle screw assembly 26 resists shear deformation of rod 22 in an anterior-posterior direction.

Vertebral construct 20 includes damping elements 60, 62 disposed within elongated opening 44 and engageable with first portion 28. The damping elements are in an opposing orientation with damping element 60 being disposed at a first end 64 of elongated opening 44 and damping element 62 being disposed at a second end 66 of elongated opening 44.

Damping element 60 is an extension damper configured for engagement with post 53 of first portion 28 in a first orientation (FIG. 4) of vertebral construct 20. Damping element 62 is a flexion damper configured for engagement with post 53 of first portion 28 in a second orientation (FIG. 5) of vertebral construct 20. One or a plurality of damping elements may be disposed within elongated opening 44.

The damping characteristics of vertebral construct 20 may be varied or adjusted according to the requirements of a particular application, such as, for example, particular flexion and extension applications. For example, damping elements may provide damping in extension and/or flexion. It is contemplated that the damping elements may be fabricated from relatively flexible materials, for example, biomedical grade polymers such as, for example, polyurethane, polyethylene or PEEK. It is further contemplated that the damping elements may have a durometer dependent upon the dampening characteristics required for a particular application. For example, in one embodiment, a low durometer polyurethane can be used for a bumper disposed at an end of a free sliding element. In another embodiment, a high durometer PEEK material can be used to correct a deformity and allow for minimal deflection.

Movement of first portion 28, for example, post 53, is limited by inner surface 42 of band portion 38 and damping elements 60, 62. As such, axial rotation, lateral bending, flexion and extension of the vertebral bodies of vertebrae V are limited by the spherical geometry of opposing spherical surfaces 54, 58, inner surface 42 and damping elements 60,62.

It is contemplated that vertebral construct 20 may include a set or kit having one of each of rod 22 and pedicle screw assemblies 24, 26, or alternatively, vertebral construct 20 can include a set or kit having a plurality of rods 22 and pedicle screw assemblies 24, 26 that may be employed for a vertebral construct spanning multiple levels and/or use with a long spinal construct.

In assembly, operation and use, vertebral construct 20 is employed with a surgical procedure for treatment of a spinal disorder affecting a section of a spine of a patient, as discussed herein. Vertebral construct 20 may also be employed with other surgical procedures. In particular, vertebral construct 20 is employed with a surgical procedure for treatment of a condition or injury, such as, for example, stenosis with posterior disc height loss, of an affected section of the spine including vertebrae V, as shown in FIGS. 6 and 7. It is contemplated that vertebral construct 20 is attached to vertebrae V for dynamic stabilization of the affected section of the spine to facilitate healing and therapeutic treatment, while providing flexion, extension and torsion capability.

In use, to treat the affected section of the spine, a medical practitioner obtains access to a surgical site including vertebra V in any appropriate manner, such as through incision and retraction of tissues. It is envisioned that vertebral construct 20 may be used in any existing surgical method or technique including open surgery, mini-open surgery, minimally invasive surgery and percutaneous surgical implantation, whereby vertebrae V is accessed through a micro-incision, or sleeve that provides a protected passageway to the area. Once access to the surgical site is obtained, the particular surgical procedure is performed for treating the spinal disorder. Vertebral construct 20 is then employed to augment the surgical treatment. Vertebral construct 20 can be delivered or implanted as a pre-assembled device or can be assembled in situ. Vertebral construct 20 may be completely or partially revised, removed or replaced, for example, replacing rod 22 and using the in-place fastening elements.

Pedicle screw assembly 24 is configured to attach band portion 38 to vertebra V₁. Pedicle screw assembly 26 is configured to attach cylinder 36 to adjacent vertebra V₂. Pilot holes are made in vertebrae V₁, V₂ for receiving screws 34, 74. Screws 34, 74 include threaded bone engaging portions 32, 72 that are inserted or otherwise connected to vertebrae V₁, V₂, according to the particular requirements of the surgical treatment. Pedicle screw assembly 24 has head 52 that is torqued to facilitate rotation of screw 34 and penetrating engagement of threads 32 with vertebrae V₁. Pedicle screw assembly 26 has head 76 with a bore, or through opening and a set screw 78, which is torqued on to cylinder 36 to attach rod 22 in place with vertebrae V.

It is envisioned that vertebral construct 20 can include two axially aligned and spaced apart rods 22 with corresponding fastening elements. Upon fixation of vertebral construct 20 with vertebrae V, the components of vertebral construct 20, described above, are configured to provide flexibility in response to movement of pedicle screw assemblies 24, 26 during flexion, extension and torsion of the spine. For example, in an unloaded state, there is no appreciable tensile, compressive or torsional loads on vertebrae V₁, V₂. In flexion, extension and/or torsion of vertebrae V caused by corresponding movement of the patient, stress and/or forces are applied to vertebral construct 20 and first portion 28 and band portion 38 react with dynamic flexibility and restriction to provide translation and multiple axial movement, including rotation, of rod 22 relative to pedicle screw assembly 24 to a plurality of orientation(s). It is contemplated that vertebral construct 20 may provide resistance, which may be increasing, decreasing, gradual, dynamic and/or static during flexion, extension and/or torsion.

For example, as shown in FIG. 4, during extension, movement of vertebrae V causes post 53 to translate in the direction of arrow A within opening 44 to engagement with damping element 60 in a first orientation. Multiple axial movement, including rotation of the surfaces of rod 22, is provided through engagement of opposing faces 46, 48 of band portion 38 with opposing spherical surfaces 54, 58, as described above. Movement of post 53 and vertebrae V is limited by inner surface 42 of band portion 38, damping elements 60, 62 and the engagement of faces 46, 48 with surfaces 54, 58.

As shown in FIG. 5, during flexion, movement of vertebrae V causes post 53 to translate in the direction of arrow A within opening 44 to engagement with damping element 62 in a second orientation. Multiple axial movement, including rotation of the surfaces of rod 22, is provided through engagement of opposing faces 46, 48 of band portion 38 with opposing spherical surfaces 54, 58, as described above. Movement of post 53 and vertebrae V is limited by inner surface 42 of band portion 38, damping elements 60, 62 and the engagement of faces 46, 48 with surfaces 54, 58.

It is envisioned that vertebral construct 20 including the alternate embodiments described may be employed in a configuration for vertebral stabilization over a plurality of intervertebral levels, including treated and untreated vertebral and intervertebral levels.

Vertebral construct 20 can be used with various bone screws, pedicle screws or multi-axial screws used in spinal surgery. It is contemplated that vertebral construct 20 may be used with pedicle screws coated with an osteoconductive material such as hydroxyapatite and/or osteoinductive agent such as a bone morphogenic protein for enhanced bony fixation to facilitate motion of the treated spinal area. The components of vertebral construct 20 can be made of radiolucent materials such as polymers. Radiomarkers may be included for identification under x-ray, fluoroscopy, CT or other imaging techniques. Metallic or ceramic radiomarkers, such as tantalum beads, tantalum pins, titanium pins, titanium endcaps and platinum wires can be used, such as being disposed at the end portions of rod 22 and/or along the length thereof.

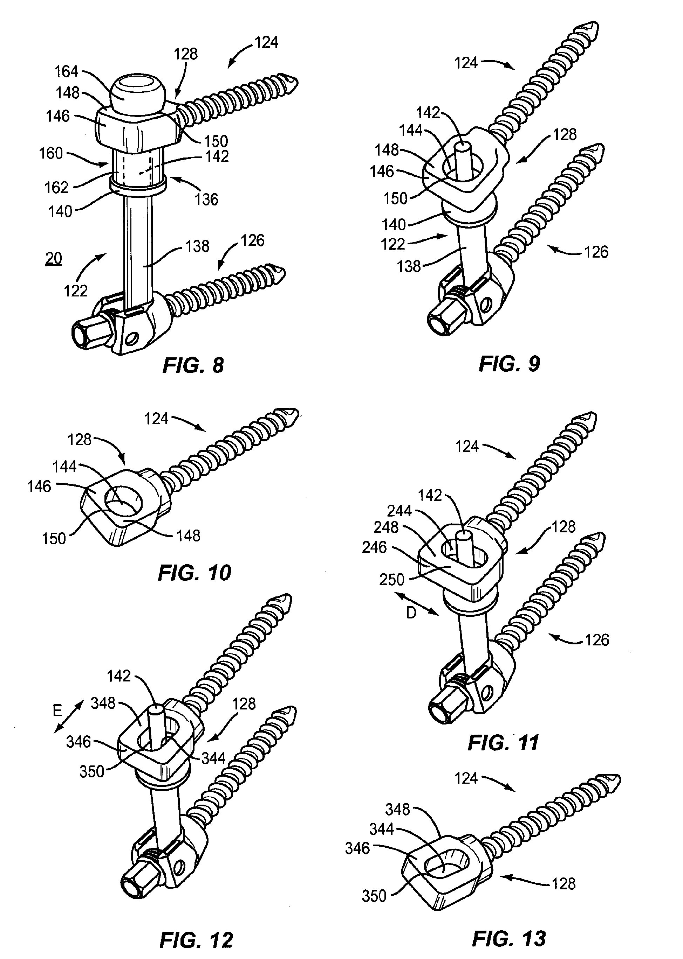

Referring to FIGS. 8-10, similar to that described with regard to FIGS. 1-7, vertebral construct 20 includes a rod 122, a first fastening element, such as, for example, a pedicle screw assembly 124 and a second fastening element, such as, for example, a pedicle screw assembly 126 that attach rod 122 to bony tissue, including vertebrae V.

Rod 122 has a first portion 136 including a flange 140 and a reduced diameter post 142. Rod 122 has a second portion, such as, for example, cylinder 138. Pedicle screw assembly 124 has a first portion 128 that defines a cavity, such as, for example, opening 144 configured to facilitate dynamic translation of post 142 therein relative to first portion 128, similar to that described above.

First portion 128 includes a band portion 146, which includes a wall 148 having an inner surface 150. Inner surface 150 defines opening 144, which is configured to facilitate dynamic translation of post 142 therein relative to first portion 128. Post 142 translates and/or toggles within opening 144. Opening 144 has a circular configuration for disposal of post 142.

Band portion 146 has a plate configuration that defines inner surface 150 configured to engage a damping element, described below. Inner surface 150 has an arcuate configuration disposed about a perimeter of opening 144. Vertebral construct 120 includes a damping element 160 disposed about post 142 and within opening 144.

Damping element 160 has a cylinder portion 162 and an enlarged spherical portion 164. Damping element 160 is engageable with inner surface 150 to facilitate multiple axis movement therebetween, similar to that described above. Spherical portion 164 movably captures first portion 128 with rod 122. Dynamic flexibility of vertebral construct 20 is provided for axial rotation, lateral bending, flexion and extension of the vertebral bodies of vertebrae and motion is limited by the geometry of inner surface 150 and damping element 160.

Referring to FIG. 11, in vertebral construct 20, similar to that described with regard to FIGS. 8-10, first portion 128 includes a band portion 246, which includes a wall 248 having an arcuate inner surface 250. Inner surface 250 defines an elliptical opening 244, which is configured to facilitate dynamic translation of post 142 therein relative to first portion 128, in the direction shown by arrows D. Post 142 translates and/or toggles within opening 244 to provide lateral and/or torsional flexibility to vertebrae. It is contemplated that band portion 246 may include one or a plurality of damping elements, similar to those described herein.

Referring to FIGS. 12-13, in vertebral construct 20, similar to that described with regard to FIGS. 8-10, first portion 128 includes a band portion 346, which includes a wall 348 having an arcuate inner surface 350. Inner surface 350 defines an elliptical opening 344, which is configured to facilitate dynamic translation of post 142 therein relative to first portion 128, in the direction shown by arrows E. Post 142 translates and/or toggles within opening 344 to provide flexibility in extension and flexion to vertebrae. It is contemplated that band portion 346 may include one or a plurality of damping elements, similar to those described herein.

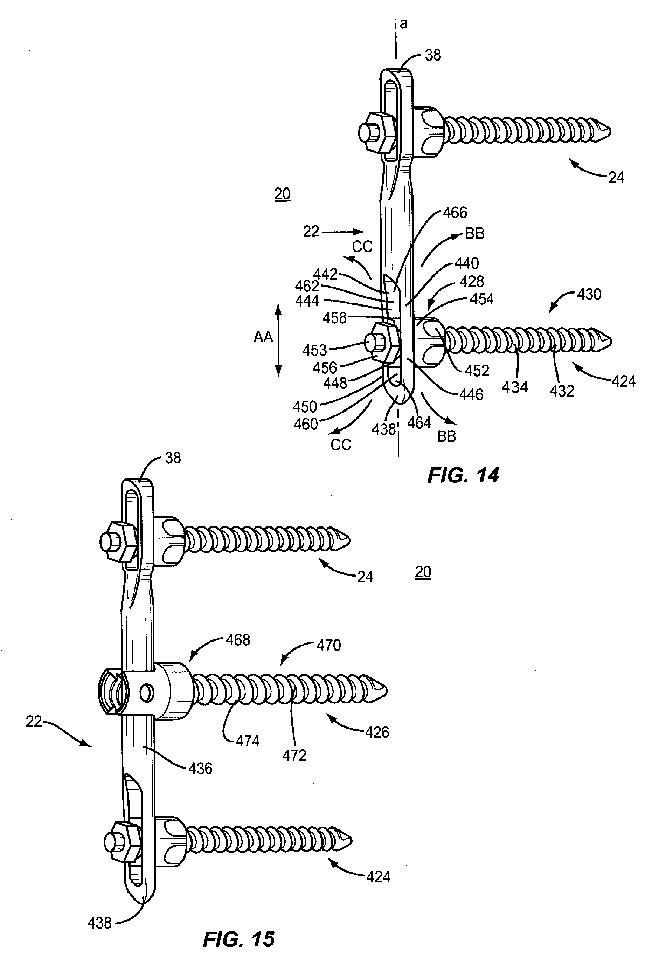

Referring to FIG. 14, vertebral construct 20 includes a rod 22 having pedicle screw assembly 24 connected and engageable with band portion 38, as described with regard to FIGS. 1-7. Vertebral construct 20 also includes a second fastening element, such as, for example, a pedicle screw assembly 424 having a first portion 428 and a second portion 430, which includes a threaded portion 432 of a screw 434. Threaded portion 432 is configured for engagement with tissue, such as, for example, fixation with vertebrae. It is contemplated that second portion 430 may be non-threaded and attached with tissue via interference or friction fit, clips and/or barbs.

Rod 22 also includes a first portion, such as, for example, a band portion 438. Band portion 438 includes a wall 440 having an inner surface 442 that defines an elongated cavity, such as, for example, an elongated opening 444 configured to facilitate dynamic translation of first portion 428 therein relative to rod 22. First portion 428 translates with elongated opening 444, in the direction of arrows AA.

Band portion 438 has an elongated plate configuration that defines a first face 446 configured to engage first portion 428 and an opposing second face 448 configured to engage an opposing portion of first portion 428. First face 446 and second face 448 are disposed about a perimeter of elongated opening 444. Band portion 438 includes bifurcated wall portions 450, which are separated and/or split to define elongated opening 444 in a configuration to capture first portion 428.

Band portion 438 and band portion 38 are disposed along a longitudinal axis *a* of rod 22. Band portion 438 may be angularly offset, perpendicular and/or staggered relative to band portion 38. It is envisioned that rod 22 may include one or a plurality of band portions. It is envisioned that band portions may be variously disposed along rod 22.

First portion 428 includes a portion of screw 434 including a head 452 having a spherical surface 454 engageable with first face 446 such that rod 22 is configured for multiple axis movement, along arrows BB, relative to pedicle screw assembly 424. It is contemplated that such multiple axis movement includes rotation, in the direction shown by arrows BB, of rod 22 relative to spherical surface 454. Head 452 has a threaded post 453 extending therefrom. First portion 428 also includes a nut 456 threaded onto post 453 to connect and movably capture rod 22 with pedicle screw assembly 424. Band portion 438 is movably attached with first portion 428 to facilitate relative translation and multiple axis movement, including rotation, therebetween, as described herein. Nut 456 is configured to prevent disassociation of rod 22 from pedicle screw assembly 424.

Nut 456 has a spherical surface 458 that is engageable with second face 448 such that rod 22 is configured for multiple axis movement, along arrows CC, relative to pedicle screw assembly 424. It is contemplated that such multiple axis movement includes rotation, in the direction shown by arrows CC, of rod 22 relative to spherical surface 458. Opposing faces 446, 448 of band portion 438 are engageable with opposing spherical surfaces 454, 458 such that rod 22 is configured for multiple axis movement, including rotation, relative to pedicle screw assembly 424. Engagement of opposing faces 446, 448 with opposing spherical surfaces 454, 458 facilitate bending of the vertebral bodies of vertebrae. Head 452 and nut 456 have a hexagonal configuration for engagement with a surgical tool to facilitate rotation and corresponding threaded fixation with vertebrae. It is contemplated that faces 446, 448 may have alternate arcuate surfaces, angled surfaces, planar surfaces and/or undulating surfaces.

Vertebral construct 20 includes damping elements 460, 462 disposed within elongated opening 444 and engageable with first portion 428. The damping elements are in an opposing orientation with damping element 460 being disposed at a first end 464 of elongated opening 444 and damping element 462 being disposed at a second end 466 of elongated opening 444.

Damping element 460 is an extension damper configured for engagement with post 453 of first portion 428 in a first orientation of vertebral construct 20. Damping element 462 is a flexion damper configured for engagement with post 453 of first portion 428 in a second orientation of vertebral construct 20. One or a plurality of damping elements may be disposed within elongated opening 444. The damping characteristics of vertebral construct 20 may be varied or adjusted according to the requirements of a particular application, such as, for example, particular flexion and extension applications.

Movement of first portion 428, for example, post 453, is limited by inner surface 442 of band portion 438 and damping elements 460, 462. As such, axial rotation, lateral bending, flexion and extension of the vertebral bodies of vertebrae are limited by the spherical geometry of opposing spherical surfaces 454, 458, inner surface 442 and damping elements 460, 462.

It is contemplated that vertebral construct 20 may include a set or kit having one of each of rod 22 including band portions 38, 438, and pedicle screw assemblies 24, 424, or alternatively, vertebral construct 20 can include a set or kit having a plurality of rods 22 including band portions 38, 438 and pedicle screw assemblies 24, 424 that may be employed for a vertebral construct spanning multiple levels and/or use with a long spinal construct.

Referring to FIG. 15, in one embodiment of vertebral construct 20, rod 22 including band portions 38, 438 and pedicle screw assemblies 24, 424, described with regard to FIG. 14, includes a pedicle screw assembly 426. Pedicle screw assembly 426 has a first portion 468 fixedly connected to a cylinder portion 436, centrally disposed along rod 22 between band portion 38 and band portion 438. Pedicle screw assembly 426 has a second portion 470 including a threaded portion 472 of a screw 474. Threaded portion 472 is configured for engagement with tissue, such as, for example, fixation with vertebrae. Rigid fixation of cylinder portion 436 to pedicle screw assembly 426 resists shear deformation of rod 22 in an anterior-posterior direction. It is envisioned that rod 22 may include one or a plurality of pedicle screw assemblies 426.

## Claims

1. A vertebral construct (20) comprising:
a first pedicle screw assembly (24;424) having a first portion (28) and a second portion (30) which includes a threaded portion (32) of a screw (34), the threaded portion (32) configured for engagement with a vertebrae (V1);
a rod (22) defining an elongated opening (44) configured to facilitate dynamic translation of the first portion (28) therein relative to the rod (22);
a second pedicle screw assembly (26;426) having a first portion (68;468) fixedly connected to the rod (22), and for attaching the rod (22) to a vertebrae (V2);
at least one damping element disposed within the elongated opening (44) and being engageable with the first portion (28) of the first pedicle screw assembly (24;424);
wherein the at least one damping element includes a pair of opposing damping elements (60,62) disposed at a first end (64) and at a second end (66) of the elongated opening (44);
wherein the rod (22) includes a band portion (38;438) having a wall (40) that defines the elongated opening (44);
wherein the band portion (38;438) has an elongated plate configuration that defines a first face (46) configured to engage the first portion (28) of the first pedicle screw assembly (24;424) and an opposing second face (48) configured to engage the first portion (28) of the first pedicle screw assembly (24;424);
wherein the first portion (28) of the first pedicle screw assembly (24;424) includes a portion of the screw (34) having a head (52);
wherein the first portion (28) of the first pedicle screw assembly (24;424) includes a nut (56);
**characterized in that**
the head (52) of the screw (34) has a spherical surface (54) engageable with the first face (46) such that the rod (22) is configured for rotation relative to the first pedicle screw assembly (24;424);
the head (52) of the screw (34) has a threaded post (53) extending therefrom;
the nut (56) is threaded onto the post (53) to connect and movably capture the rod (22);
the nut (56) has a spherical surface (58) that is engageable with the second face (48) such that the rod (22) is configured for rotation relative to the first pedicle screw assembly (24);
multiple axis movement, including rotation of the surfaces of the rod (22), is provided through engagement of the opposing faces (46,48) of the band portion (38;438) with opposing spherical surfaces (54;58); and
upon fixation of the components of the vertebral construct (20) with the vertebrae (V1, V2), the components of the vertebral construct (20) are configured to provide flexibility in response to movement of the pedicle screw assemblies (24, 26) during flexion, extension and torsion of the spine, wherein, during extension, movement of the vertebrae (V1, V2) causes the post (53) to translate in a direction (arrow A) within the opening (44) to engage with the damping element (60) disposed at the first end (64) in a first orientation, and wherein, during flexion, movement of the vertebrae (V1, V2) causes the post (53) to translate in said direction (arrow A) within the opening (44) to engage with the damping element (62) disposed at the second end (66) in a second orientation.

2. A vertebral construct (20) according to Claim 1, wherein the rod (22) includes a bifurcated wall portion (50;450) defining the elongated opening (44;444) as a through opening such that the wall portions are configured to capture the first portion (28;428) of the first pedicle screw assembly (24;424).

## Patentansprüche

1. Eine Wirbelkonstruktion (20), aufweisend:
eine erste Pedikelschraubeneinrichtung (24, 424) mit einem ersten Abschnitt (28) und einem zweiten Abschnitt (30), welcher einen Gewindeabschnitt (32) einer Schraube (34) aufweist, wobei der Gewindeabschnitt (32) konfiguriert ist zum In-Eingriff-Stehen mit einem Wirbel (V1),
einen Stab (22), welcher eine längliche Öffnung (44) definiert, die konfiguriert ist, sodass in ihr ein dynamisches Translationsverschieben des ersten Abschnitts (28) relativ zu dem Stab (22) erleichtert ist,
eine zweite Pedikelschraubeneinrichtung (26, 426) mit einem fest mit dem Stab (22) verbundenen ersten Abschnitt (68, 468), und zum Befestigen des Stabs (22) an einem Wirbel (V2),
mindestens ein Dämpfungselement, welches in der länglichen Öffnung (44) angeordnet ist und mit dem ersten Abschnitt (28) der ersten Pedikelschraubeneinrichtung (24, 424) in Eingriff gebracht werden kann,
wobei das mindestens eine Dämpfungselement aufweist ein Paar gegenüberliegender Dämpfungselemente (60, 62), welche an einem ersten Ende (64) und an einem zweiten Ende (66) der länglichen Öffnung (44) angeordnet sind,
wobei der Stab (22) aufweist einen Bandabschnitt (38, 438) mit einer Wand (40), welche die längliche Öffnung (44) definiert,
wobei der Bandabschnitt (38, 438) aufweist eine längliche Plattenkonfiguration, welche eine erste Fläche (46) definiert, die konfiguriert ist, um mit dem ersten Abschnitt (28) der ersten Pedikelschraubeneinrichtung (24, 424) in Eingriff zu stehen, und eine entgegengesetzte zweite Fläche (48), die konfiguriert ist, um mit dem ersten Abschnitt (28) der ersten Pedikelschraubeneinrichtung (24, 424) in Eingriff zu stehen,
wobei der erste Abschnitt (28) der ersten Pedikelschraubeneinrichtung (24, 424) aufweist einen Abschnitt der Schraube (34), der einen Kopf (52) aufweist,
wobei der erste Abschnitt (28) der ersten Pedikelschraubeneinrichtung (24, 424) eine Mutter (56) aufweist,
**dadurch gekennzeichnet, dass**
der Kopf (52) der Schraube (34) eine sphärische Fläche (54) aufweist, welche mit der ersten Fläche (46) in Eingriff gebracht werden kann, sodass der Stab (22) zum Drehen relativ zu der ersten Pedikelschraubeneinrichtung (24, 424) konfiguriert ist,
der Kopf (52) der Schraube (34) einen sich davon ausgehend erstreckenden Gewindeschaft (53) aufweist,
die Mutter (56) auf den Schaft (53) geschraubt ist zum Verbinden und bewegbaren Halten des Stabs (22),
die Mutter (56) eine sphärische Fläche (58) aufweist, welche mit der zweiten Fläche (48) in Eingriff gebracht werden kann, sodass der Stab (22) zum Drehen relativ zu der ersten Pedikelschraubeneinrichtung (24) konfiguriert ist,
eine mehrachsige Bewegung, inklusive eines Drehens der Flächen des Stabs (22), ermöglicht ist durch den Eingriff der entgegengesetzten Flächen (46, 48) des Bandabschnitts (38, 438) mit den gegenüberliegenden sphärischen Flächen (54, 58), und
auf das Fixieren der Komponenten der Wirbelkonstruktion (20) mit den Wirbeln (V1, V2), die Komponenten der Wirbelkonstruktion (20) konfiguriert sind, um Flexibilität bereitzustellen in Reaktion auf eine Bewegung der Pedikelschraubeneinrichtungen (24, 26) bei Flexion, Extension und Torsion der Wirbelsäule, wobei, während einer Extension, eine Bewegung der Wirbel (V1, V2) den Schaft (53) veranlasst, sich innerhalb der Öffnung (44) in eine Richtung (Pfeil A) translationszubewegen, um mit dem an dem ersten Ende (64) angeordneten Dämpfungselement (60) in einer ersten Orientierung in Eingriff zu kommen, und wobei, während einer Flexion, eine Bewegung der Wirbel (V1, V2) den Schaft (53) veranlasst, sich innerhalb der Öffnung (44) in dieser Richtung (Pfeil A) translationszubewegen, um mit dem an dem zweiten Ende (66) angeordneten Dämpfungselement (62) in einer zweiten Orientierung in Eingriff zu kommen.

2. Eine Wirbelkonstruktion (20) gemäß Anspruch 1, wobei der Stab (22) aufweist einen gegabelten Wandabschnitt (50, 450), welcher die längliche Öffnung (44, 444) als eine Durchgangsöffnung definiert, sodass die Wandabschnitte konfiguriert sind, um den ersten Abschnitt (28, 428) der ersten Pedikelschraubeneinrichtung (24, 424) zu halten.

## Revendications

1. Construction vertébrale (20) comprenant :
un premier ensemble de vis pédiculaire (24 ; 424) ayant une première partie (28) et une seconde partie (30) qui comprend une partie filetée (32) d'une vis (34), la partie filetée (32) étant configurée pour la mise en prise avec une vertèbre (V1) ;
une tige (22) définissant une ouverture allongée (44) configurée pour faciliter la translation dynamique de la première partie (28) à l'intérieur de cette dernière par rapport à la tige (22) ;
un second ensemble de vis pédiculaire (26 ; 426) ayant une première partie (68 ; 468) fixement raccordée à la tige (22), et pour fixer la tige (22) à une vertèbre (V2) ;
au moins un élément d'amortissement disposé à l'intérieur de l'ouverture allongée (44) et pouvant se mettre en prise avec la première partie (28) du premier ensemble de vis pédiculaire (24 ; 424) ;
dans laquelle le au moins un élément d'amortissement comprend une paire d'éléments d'amortissement (60, 62) opposés disposés au niveau d'une première extrémité (64) et au niveau d'une seconde extrémité (66) de l'ouverture allongée (44);
dans laquelle la tige (22) comprend une partie de bande (38 ; 438) ayant une paroi (40) qui définit l'ouverture allongée (44) ;
dans laquelle la partie de bande (38 ; 438) a une configuration de plaque allongée qui définit une première face (46) configurée pour mettre en prise la première partie (28) du premier ensemble de vis pédiculaire (24 ; 424) et une seconde face (48) opposée configurée pour mettre en prise la première partie (28) du premier ensemble de vis pédiculaire (24 ; 424) ;
dans laquelle la première partie (28) du premier ensemble de vis pédiculaire (24 ; 424) comprend une partie de la vis (34) ayant une tête (52) ;
dans laquelle la première partie (28) du premier ensemble de vis pédiculaire (24 ; 424) comprend un écrou (56);
**caractérisée en ce que**
la tête (52) de la vis (34) a une surface sphérique (54) pouvant se mettre en prise avec la première face (46) de sorte que la tige (22) est configurée pour tourner par rapport au premier ensemble de vis pédiculaire (24 ; 424);
la tête (52) de la vis (34) a un montant fileté (53) s'étendant à partir de cette dernière ;
l'écrou (56) est vissé sur le montant (53) pour raccorder et capturer de manière mobile la tige (22) ;
l'écrou (56) a une surface sphérique (58) qui peut se mettre en prise avec la seconde face (48) de sorte que la tige (22) est configurée pour tourner par rapport au premier ensemble de vis pédiculaire (24) ;
un mouvement multiaxial, comprenant la rotation des surfaces de la tige (22), est fourni par le biais de la mise en prise des faces opposées (46, 48) de la partie de bande (38 ; 438) avec des surfaces sphériques (54 ; 58) opposées ; et
suite à la fixation des composants de la construction vertébrale (20) avec les vertèbres (V1, V2), les composants de la construction vertébrale (20) sont configurés pour fournir la flexibilité en réponse au mouvement des ensembles de vis pédiculaire (24, 26) pendant la flexion, l'extension et la torsion de la colonne vertébrale, dans lesquels, pendant l'extension, le mouvement des vertèbres (V1, V2) provoque la translation du montant (53) dans une direction (flèche A) à l'intérieur de l'ouverture (44) pour se mettre en prise avec l'élément d'amortissement (60) disposé au niveau de la première extrémité (64) dans une première orientation, et dans lesquels, pendant la flexion, le mouvement des vertèbres (V1, V2) provoque la translation du montant (53) dans ladite direction (flèche A) à l'intérieur de l'ouverture (44) afin de se mettre en prise avec l'élément d'amortissement (62) disposé au niveau de la seconde extrémité (66) dans une seconde orientation.

2. Construction vertébrale (20) selon la revendication 1, dans laquelle la tige (22) comprend une partie de paroi bifurquée (50 ; 450) définissant l'ouverture allongée (44 ; 444) sous la forme d'une ouverture débouchante de sorte que les parties de paroi sont configurées pour capturer la première partie (28 ; 428) du premier ensemble de vis pédiculaire (24 ; 424).
